(19) Europäisches Patentamt / European Patent Office / Office européen des brevets

(11) **EP 3 903 801 A1**

(12) **EUROPEAN PATENT APPLICATION**

(43) Date of publication:
**03.11.2021 Bulletin 2021/44**

(51) Int Cl.:
**A61K 36/752** (2006.01)    **A61P 3/04** (2006.01)
**A61P 3/06** (2006.01)

(21) Application number: **21167967.5**

(22) Date of filing: **13.04.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.04.2020 IT 202000008797**

(71) Applicant: **Naturextralab S.r.l.**
**87040 Mendicino (IT)**

(72) Inventor: **Statti, Giancarlo**
**87036 RENDE (IT)**

(74) Representative: **Perrotta, Aldo**
**Ing. Aldo Perrotta**
**Corso Umberto 81**
**88068 Soverato (IT)**

(54) **FORMULATION COMPRISING CITRUS BERGAMIA L. AND FORTUNELLA JAPONICA EXTRACTS AND ITS USE TO LIMIT PANCREATIC LIPASE**

(57)    Formulation based on plant extracts comprising extracts of Citrus bergamia L. and Fortunella japonica where said formulation provides a formulation with an 80/20 mixture, that is, with 80% of Citrus bergamia L. and 20% of Fortunella japonica L and where said formulation is used to limit pancreatic lipase.

EP 3 903 801 A1

**Description**

**Technical field of the invention**

This invention concerns a formulation based on plants extracts and the use of the mentioned formulation based on plants extract to limit pancreatic lipase in order to reduce the hydrolysis of fat.

**State of the art**

Lipids are fundamental components of nutrition, but an increased supply contributes to the onset of obesity and other related diseases in the long term.

According to the World Health Organization, obesity is a global epidemic risk and is based on analysis of body mass index data (BMI) (WHO, 1998). This is a complex disease whose cause is to be sought in a series of concomitant factors: genetic, dietary and environmental that, together with wrong lifestyles, may lead to a chronic positive energy balance and, consequently, to an increase in fat mass (Mohamed et al., 2014).

Obesity is closely related to increased development of metabolic disorders (diabetes, hypertension), cardiovascular disease and other chronic conditions. It can be iatrogenic or secondary to pharmacological treatments (antipsychotic drugs, antidepressants, antiepileptic drugs, steroids, insulin) or to pathologies (Cushing's syndrome, hypothyroidism, hypothalamic defects), (Derdemezis et al., 2011; Mohamed et al., 2014; Singla et al., 2010).

In cases of severe obesity, drug therapy is associated with diet and exercise. Among the most common antiobesity drugs, Orlistat is of great importance: it's a semi-synthetic derivative of lipostatin. This is an inhibitor of gastric and pancreatic lipases, which are enzymes that play a key role in the digestion of food fats (Al-Suwailem et al, 2006). This drug acts on the enzymes present within the gastrointestinal tract, covalently binding to the serine residue of the active site of the enzyme, thereby inhibiting biological activity (Al-Suwailem et al, 2006).

As mentioned above, Orlistat is one of the most widely used drugs in the treatment of obesity. But it is also responsible for a wide variety of side effects such as steatorrhoea, swelling, urgency and fecal incontinence. Between 16% and 40% of patients treated with Orlistat has at least one of these side effects, whereas fecal incontinence is definitely the most limiting side effect of the therapy (Drew et al, 2007; Padwal et al 2003).

The pancreatic lipase, triacylglycerol hydrolases, is the enzyme responsible for the absorption of triglycerides, since it acts by hydrolysing them to monoacylglycerols and fat acids. Reduced hydrolysis of fats allows a reduced rate of their absorption (Slanc et al., 2009).

**Evaluation of the inibition of the pancreatic lipase enzyme activity**

In order to assess the inhibition of the pancreatic lipase enzyme activity, a test has been developed.

The test has been conducted in vitro according to protocols validated by the scientific literature and each analysis was conducted in triplicate to obtain the mean value. The inhibition of enzyme activity was spectrophotometrically evaluated, using p-Nitrophenyl octanoate (p-NPO), chromogenic ester, as substrate. Lipase has a high affinity to NPO, so as to hydrolyse the ester bond between the p-nitrophenol and the fat acid molecule; maximum activity was observed in alkaline conditions (Yeoh et al, 1986; Mrdakovic et al, 2008). An aqueous enzyme solution (1 mg/ml) has been prepared from crude porcine pancreas type II (Conforti et al., 2012). A solution of $27 \mu l$ di NPO in 19,973 ml of dimethyl sulfoxide (DMSO) has been prepared. The composition of the reaction mixture is the following: 25 $\mu l$ NPO, 1 ml Tris-Hcl buffer (pH = 8,0), 25 $\mu l$ extract (concentration 430,00 mg/ml; 322,50 mg/ml; 215,00 mg/ml; 107,50 mg/ml; 43,00 mg/ml; 21,50 mg/ml; 10,75 mg/ml; 5,75 mg/ml; 2,687 mg/ml; 1,345 mg/ml; 0,67 mg/ml) and 25 $\mu l$ enzymatic solution.

The mixture has been incubated at 37 ° C. In the check, the same volume of DMSO was added instead of the extract. Orlistat was used for positive control. Inhibitory activity was assessed using the following formula:

$$\text{Lipase inhibitory activity (\%)} = [1 - (ABS_{NPO} - ABS_{DMSO}) / ABS_0] \times 100$$

Where: $ABS_0$ is the absorbance of enzyme activity at 100% of the substrate (control).

Evaluation of the tests

The concentration of a drug, antibody or toxicant which induces a response halfway between the baseline and maximum after a specified exposure time **is defined Half maximal effective concentration ( EC $_{50}$ )**. It is commonly used as a measure of a drug's potency. When the measured activity has an inhibiting effect it is called **IC$_{50}$**.

The biological activity of inhibition of pancreatic lipase of the following natural extracts has been assessed.

These extracts are known for their inhibitory activity against pancreatic lipase and $IC_{50}$ value of each of them has been measured:

*Citrus bergamia* L.: $IC_{50}$ 0,52 ± 0,13 mg/ml
*Fortunella japonica* L: $IC_{50}$ 0,60 ± 0,07 mg/ml

Different formulations based on the two extracts have been prepared and the $IC_{50}$ values of each formulation have been determined.
Mixture 50/50: $IC_{50}$ 0,55 ± 0,04 mg/ml
Formulation with 50% *Citrus bergamia* L. and 50% *Fortunella japonica* L
Mixture 70/30: $IC_{50}$ 0,79 ± 0,07 mg/ml
Formulation with 70% *Citrus bergamia* L. and 30% *Fortunella japonica* L
Mixture 80/20: $IC_{50}$ 0,47 ± 0,05 mg/ml
Formulation with 80% *Citrus bergamia* L. and 20% *Fortunella japonica* L
Mixture 90/10: $IC_{50}$ 0,62 ± 0,02 mg/ml
Formulation with 90% *Citrus bergamia* L. and 10% *Fortunella japonica* L
It has been observed that the formulation with the mixture 50/50, that is 50% *Citrus bergamia* L. and 50% and *Fortunella japonica* L shows an $IC_{50}$ value with dilution between those of the single extracts, whereas the formulations with mixture 70/30 and 90/10 show an $IC_{50}$ value with higher concentrations than those of the single extracts.
Eventually, the mixture 80/20, that is 80% *Citrus bergamia* L. and 20% *Fortunella japonica* L demonstrates an $IC_{50}$ with lower concentration than those of the single extracts.
Regarding the formulation based on plant extract with mixture 80/20, that is 80% Citrus bergamia L. and 20% Fortunella japonica L it has been observed a synergic effect of two extracts of high inhibitory activity against pancreatic lipase.
The synergism is obtained when, from the interaction between two plants, we get a sum of effects, which is a higher effect with respect to the effects of the single plants.

Bibliography:

Al-Suwailem K., Al-Tamimi A.S., Al-Omar M.A., and. Al-Suhibani M.S. Safety and Mechanism of Action of Orlistat (Tetrahydrolipstatin) as the First Local Antiobesity Drug, 2006, Journal of Applied Sciences Research, C(): CC-CC, 2006.

Conforti F., Perri V., Menichini F., Marrelli M., Uzunov D., Statti G.A., Menichini F. Wild Mediterranean dietary plants as inhibitors of pancreatic lipase. Phytotherapy Research, 2012, 26, 4, 600-604.

Derdemezis C. S., Voulgari P. V., Drosos A. A. Kiortsis D. N. Obesity adipose tissue and rheumatoid arthritis: coincidence or more complex relationship? Clinical and Experimental Rheumatology, 2011, 29, 712-727.

Drew B.S., Dixon A.F., Dixon J.B. Obesity management: Update on orlistat, Vascular Health Risk Management, 2007, 3, 6, 817-821.

Mohamed G. A., Ibrahim S. R.M., Elkhayat E. S., El Dine R. S. Natural anti-obesity agents, Bulletin of Faculty of Pharmacy, Cairo University 2014, 52, 269-284.

Mrdakovic M., Lazarevi_C.J., Peri_C., Mataruga V., Ilijin L., and Vlahovi C.M. Partial Characterization of a Lipase from Gypsy Moth (Lymantria dispar L.) Larval Midgut, 2008, Folia biologica, Kraków, 56, 1-2.

Padwal R., Li S.K., Lau D.C. Long-term pharmacotherapy for obesity and overweight. Cochrane Database Systematic Review, 2003, 4:CD004094.

Singla P., Bardoloi A., Parkash A. A. Metabolic effects of obesity: a review. World Journal of Diabetes 2010, 1, 76-88.

Slanc P., Doljak B., Kreft S., Lunder M., Janeš D., Štrukelj B. Screening of selected food and medicinal plant extracts for pancreatic lipase inhibition, Phytotherapy Research, 2009, 23, 874-877.

World Health Organization. Obesity: preventing and managing the global epidemic report of a WHO consultation on obesity. WHO: Geneva, Switzerland; 1998.

Yeoh H.H., Wong F.M., Lin G. Screening for fungal lipases using chromogenic lipid substrates, 1986, Mycologia, 78:298.

**Claims**

1. Formulation based on plant extracts comprising extracts of Citrus bergamia L. and Fortunella japonica **characterized in that** said formulation provides a formulation with an 80/20 mixture, that is, with 80% of Citrus bergamia L. and 20% of Fortunella japonica L

2. Formulation based on plant extracts extracts comprising extracts of Citrus bergamia L. and Fortunella japonica **characterized in that** said formulation is used to limit pancreatic lipase and provides a formulation with an 80/20 mixture, that is, with 80% of Citrus bergamia L. and 20% of Fortunella japonica L

Europäisches
Patentamt

European
Patent Office

Office européen
des brevets

**EUROPEAN SEARCH REPORT**

Application Number

EP 21 16 7967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE WPI<br>Week 200339<br>Thomson Scientific, London, GB;<br>AN 2003-407295<br>XP002801763,<br>& JP 2002 326947 A (KAO CORP)<br>15 November 2002 (2002-11-15)<br>* abstract * | 1,2 | INV.<br>A61K36/752<br>A61P3/04<br>A61P3/06 |
| A | US 2009/274782 A1 (FUJIKAWA TAKAHIKO [JP] ET AL) 5 November 2009 (2009-11-05)<br>* example 1 *<br>* paragraph [0045] - paragraph [0046] *<br>* claims * | 1,2 | |
| A | DATABASE WPI<br>Week 201609<br>Thomson Scientific, London, GB;<br>AN 2016-064649<br>XP002801764,<br>& CN 104 815 138 A (JUZHIHUI TECHNOLOGY INFORMATION CONSULTI)<br>5 August 2015 (2015-08-05)<br>* abstract * | 1,2 | |
| A | DATABASE WPI<br>Week 200879<br>Thomson Scientific, London, GB;<br>AN 2008-N62387<br>XP002801765,<br>& JP 2008 253256 A (KORITSU DAIGAKU HOJIN OOSAKA FURITSU DAIGAKU)<br>23 October 2008 (2008-10-23)<br>* abstract * | 1,2 | |
| A | CN 105 031 537 A (HUO JUNMEI)<br>11 November 2015 (2015-11-11)<br>* abstract * | 1,2 | |

TECHNICAL FIELDS SEARCHED (IPC)

A61K
A61P

-/--

The present search report has been drawn up for all claims

| Place of search | Date of completion of the search | Examiner |
|---|---|---|
| Munich | 20 September 2021 | Fey-Lamprecht, F |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 21 16 7967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE WPI<br>Week 201849<br>Thomson Scientific, London, GB;<br>AN 2018-42381A<br>XP002801766,<br>& CN 108 066 549 A (JIANG Q)<br>25 May 2018 (2018-05-25)<br>* abstract *<br>----- | 1 | |

TECHNICAL FIELDS
SEARCHED     (IPC)

The present search report has been drawn up for all claims

| Place of search | Date of completion of the search | Examiner |
|---|---|---|
| Munich | 20 September 2021 | Fey-Lamprecht, F |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 7967

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2021

| Patent document cited in search report | | Publication date | Patent family member(s) | | Publication date |
|---|---|---|---|---|---|
| JP 2002326947 | A | 15-11-2002 | JP | 3953847 B2 | 08-08-2007 |
| | | | JP | 2002326947 A | 15-11-2002 |
| US 2009274782 | A1 | 05-11-2009 | JP | 2009269839 A | 19-11-2009 |
| | | | US | 2009274782 A1 | 05-11-2009 |
| CN 104815138 | A | 05-08-2015 | NONE | | |
| JP 2008253256 | A | 23-10-2008 | JP | 5699416 B2 | 08-04-2015 |
| | | | JP | 2008253256 A | 23-10-2008 |
| CN 105031537 | A | 11-11-2015 | NONE | | |
| CN 108066549 | A | 25-05-2018 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AL-SUWAILEM K. ; AL-TAMIMI A.S. ; AL-OMAR M.A. ; AL-SUHIBANI M.S.** Safety and Mechanism of Action of Orlistat (Tetrahydrolipstatin) as the First Local Antiobesity Drug. *Journal of Applied Sciences Research,* 2006

- **CONFORTI F. ; PERRI V. ; MENICHINI F. ; MARRELLI M. ; UZUNOV D. ; STATTI G.A. ; MENICHINI F.** Wild Mediterranean dietary plants as inhibitors of pancreatic lipase. *Phytotherapy Research,* 2012, vol. 26 (4), 600-604

- **DERDEMEZIS C. S. ; VOULGARI P. V. ; DROSOS A. A. ; KIORTSIS D. N.** Obesity adipose tissue and rheumatoid arthritis: coincidence or more complex relationship?. *Clinical and Experimental Rheumatology,* 2011, vol. 29, 712-727

- **DREW B.S. ; DIXON A.F. ; DIXON J.B.** Obesity management: Update on orlistat. *Vascular Health Risk Management,* 2007, vol. 3 (6), 817-821

- **MOHAMED G. A. ; IBRAHIM S. R.M. ; ELKHAYAT E. S. ; EI DINE R. S.** Natural anti-obesity agents, Bulletin of Faculty of Pharmacy. Cairo University, 2014, vol. 52, 269-284

- **MRDAKOVIC M. ; LAZAREVI_C.J. ; PERI_C. ; MATARUGA V. ; ILIJIN L. ; VLAHOVI C.M.** Partial Characterization of a Lipase from Gypsy Moth (Lymantria dispar L.) Larval Midgut. *Folia biologica,* 2008, vol. 56, 1-2

- **PADWAL R. ; LI S.K. ; LAU D.C.** Long-term pharmacotherapy for obesity and overweight. *Cochrane Database Systematic Review,* 2003, vol. 4

- **SINGLA P. ; BARDOLOI A. ; PARKASH A. A.** Metabolic effects of obesity: a review. *World Journal of Diabetes,* 2010, vol. 1, 76-88

- **SLANC P. ; DOLJAK B. ; KREFT S. ; LUNDER M. ; JANES̆ D. ; S̆TRUKELJ B.** Screening of selected food and medicinal plant extracts for pancreatic lipase inhibition. *Phytotherapy Research,* 2009, vol. 23, 874-877

- **YEOH H.H. ; WONG F.M. ; LIN G.** Screening for fungal lipases using chromogenic lipid substrates. *Mycologia,* 1986, vol. 78, 298